# FASCICULE DE BREVET EUROPEEN

(11) **EP 0 650 056 B1**
(45) Date de publication et mention de la délivrance du brevet: **05.01.2000**
(21) Numéro de dépôt: 94402361.3
(22) Date de dépôt: 20.10.1994
(51) Int. Cl.: G01N 35/00, G21F 7/06, B65G 51/00

(54) **Chaîne d'analyses automatisée**
Automatische Aufeinanderfolge von Analysevorrichtungen
System for automatic analyses

(30) Priorité: 22.10.1993 FR 9312626
(43) Date de publication de la demande: 26.04.1995
(73) Titulaire: COMPAGNIE GENERALE DES MATIERES NUCLEAIRES, 78140 Velizy Villacoublay (FR)
(72) Inventeur: Besnier, Joseph, F-50440 Beaumont-Hague (FR)
(74) Mandataire: Dubois-Chabert, Guy

(56) Documents cités:
- EP-A- 0 003 936
- EP-A- 0 286 536
- EP-A- 0 509 919
- EP-A- 0 522 959
- EP-A- 0 557 828
- GB-A- 2 165 048
- IEEE TRANSACTIONS ON INDUSTRY APPLICATIONS, vol.28, no.4, Août 1992, NEW YORK US pages 938 - 944 GECKS 'robotics an efficient tool for laboratory automation'
- PATENT ABSTRACTS OF JAPAN vol. 4, no. 10 (P-022) 8 Août 1980 & JP-A-55 066 758 (TOSHIBA) 20 Mai 1980
- PATENT ABSTRACTS OF JAPAN vol. 17, no. 157 (P-1511) 26 Mars 1993 & JP-A-04 323 562 (MITSUBISHI) 12 Novembre 1992
- CHEMOMETRICS, vol.21, no.2-3, Décembre 1993, NL pages 207 - 214 BEUGELSDIJK ET AL. 'standard laboratory module'

## Description

L'invention concerne une chaîne d'analyses conçue pour effectuer, de façon automatisée, des analyses telles que des analyses chimiques sur des échantillons liquides contenus dans des cruchons.

Une telle chaîne d'analyses peut notamment être utilisée dans l'industrie nucléaire pour contrôler périodiquement les caractéristiques de certains produits dans une usine de fabrication ou de retraitement, sans présenter de risque pour le personnel ni pour l'environnement.

Le document FR-A-2 675 582 décrit une installation automatisée permettant de prélever des échantillons liquides en différents points d'une usine nucléaire, d'introduire ces échantillons dans des cruchons et de transférer ces cruchons dans des boîtes d'analyses par un réseau de transfert pneumatique piloté par une unité centrale de commande.

Le document FR-A-2 675 902 décrit un dispositif susceptible d'être utilisé dans une telle installation automatisée, pour prélever des échantillons liquides et les introduire automatiquement dans des cruchons fermés permettant leur transfert vers des boîtes d'analyses.

Les installations existantes, illustrées notamment par les documents précités, permettent d'effectuer de façon automatique les prélèvements ainsi que le transfert des cruchons dans les boîtes d'analyses. Cependant, les différentes opérations qui sont ensuite effectuées à l'intérieur des boîtes d'analyses sont réalisées manuellement, à l'aide de pinces de télémanipulation, par des opérateurs placés derrière des hublots équipant les boîtes d'analyses. La protection du personnel est assurée à la fois par une étanchéité complète des boîtes d'analyses, qui empêche la migration de la contamination vers l'extérieur, et par des blindages qui protègent le personnel vis-à-vis de l'irradiation.

L'exécution manuelle des différentes opérations qui doivent être réalisées dans les boîtes d'analyses présente cependant de nombreux inconvénients qui vont à présent être exposés.

Un premier inconvénient concerne l'identification des cruchons. En effet, cette identification est faite par l'opérateur à travers un hublot de forte épaisseur et donc très déformant. De plus, cette identification concerne des cruchons qui chutent en vrac dans le fond de la boîte d'analyses, à leur arrivée dans celle-ci. Ce problème entraîne un risque d'erreur et même de perte de certains cruchons. Cela conduit fréquemment les opérateurs à demander des cruchons supplémentaires et se traduit donc par un accroissement des déchets liquides et solides.

Un autre inconvénient concerne le débouchage des cruchons à l'aide des pinces de télémanipulation. Cette opération est particulièrement délicate et conduit à une perte de temps et à un risque de déversement du contenu dans le fond de la boîte d'analyses.

En outre, il est strictement impossible de reboucher les cruchons à l'aide de pinces dans la boîte d'analyses. Par conséquent, la récupération des surplus d'échantillons liquides est pratiquement impossible ou ne pourrait être envisagée qu'à condition de créer un réseau gravitaire de collecte coûteux et délicat à concevoir dans le cadre du matériel existant.

D'autres inconvénients découlent de l'utilisation d'une pipette de laboratoire classique pour effectuer dans les cruchons les prélèvements nécessaires aux analyses. Cette technique nécessite en effet de prélever, pour chaque analyse, une quantité de liquide relativement importante (au moins 2 ml), ce qui conduit à un volume d'effluents liquides important. Par ailleurs, il n'est pratiquement pas possible de faire plusieurs analyses à partir d'un même cruchon, ce qui accroît aussi considérablement les déchets solides et liquides. En outre, la quantité de réactifs chimiques utilisée est importante, ce qui accroît le coût d'exploitation. Enfin, les réactifs chimiques analytiques constituent des ions gênants dont la quantité importante utilisée est très pénalisante vis-à-vis du traitement des effluents et/ou d'un recyclage éventuel.

Le document EP-A-0 557 828 décrit une installation permettant de préparer, de façon automatisée, des échantillons liquides contenus dans des flacons initialement fermés par des capuchons vissés, en vue de leur analyse ultérieure. L'installation comprend un poste d'ouverture, où le capuchon est dévissé, et un agitateur, au niveau duquel un réactif est versé dans le flacon. Il comprend aussi un bras de manutention permettant de déplacer les flacons contenant les échantillons entre les différents postes. Le bras permet aussi de manipuler un flacon de réactif, un porte-aiguille, ainsi qu'une aiguille.

L'invention a précisément pour objet une chaîne d'analyses dont la conception permette à la fois de diminuer la quantité de déchets solides, de diminuer la quantité d'effluents liquides et d'accroître la productivité, en conservant les boîtes d'analyses existantes et en préservant le double confinement radiologique de ces boîtes.

Conformément à l'invention, ce résultat est obtenu au moyen d'une chaîne d'analyses automatisée conforme à la revendication 1.

L'automatisation des opérations effectuées à l'intérieur de chaque boîte d'analyses permet de supprimer tous les inconvénients qui découlent de l'exécution manuelle de ces opérations dans les installations existantes.

Il est à noter que le bouchage et le débouchage automatisés des cruchons sont facilités en utilisant des cruchons comportant des bouchons vissés.

Grâce aux moyens de stockage des cruchons et aux moyens de préhension, il est possible de stocker dans chaque boîte d'analyses aussi bien des cruchons destinés à subir des analyses à l'intérieur de cette boîte que des cruchons destinés à être directement réexpédiés vers des appareils extérieurs.

Avantageusement, chaque boîte d'analyses comporte de plus des moyens de pesage automatique des cruchons à leur arrivée dans cette boîte d'analyses et des moyens de tri, pilotés par l'unité centrale de commande en réponse à des signaux délivrés par les moyens de pesage, aptes à introduire automatiquement les cruchons pleins dans les moyens de stockage et à éliminer les cruchons vides, par exemple en les faisant chuter dans le fond de la boîte d'analyses ou, de préférence, en les réexpédiant en l'état vers une unité de destruction des cruchons.

La chaîne d'analyses peut également comprendre au moins un appareil de mesure non destructive tel qu'un appareil de mesure physique, relié par le réseau de transfert pneumatique aux premiers moyens pour réexpédier les cruchons et à un poste d'alimentation directe des moyens de stockage équipant chaque boîte d'analyses.

Un appareillage de recyclage de liquide restant dans les cruchons est avantageusement prévu, cet appareillage étant relié par le réseau de transfert pneumatique aux premiers moyens pour réexpédier les cruchons.

De préférence, le réseau de transfert pneumatique comprend des moyens d'aiguillage des cruchons, aptes à diriger alternativement les cruchons en provenance des premiers moyens pour réexpédier les cruchons vers l'appareillage de recyclage et vers les appareils de mesures non destructives, et aptes à diriger alternativement les cruchons en provenance des appareils de mesures non destructives vers le poste d'alimentation directe et vers l'appareillage de recyclage.

Grâce à ces caractéristiques, il est possible d'effectuer les analyses et les mesures non destructives dans cet ordre ou dans l'ordre inverse pour chacun des cruchons.

La chaîne d'analyses peut également comprendre au moins une boîte d'analyses manuelles comportant des moyens d'ouverture et de fermeture des cruchons et des deuxièmes moyens pour réexpédier les cruchons dans le réseau de transfert pneumatique.

Dans ce dernier cas, les moyens d'aiguillage sont également aptes à diriger les cruchons en provenance des premiers moyens pour réexpédier les cruchons vers la boîte d'analyses manuelles.

De préférence, la boîte d'analyses manuelles comporte de plus des moyens de lavage et de rinçage des cruchons.

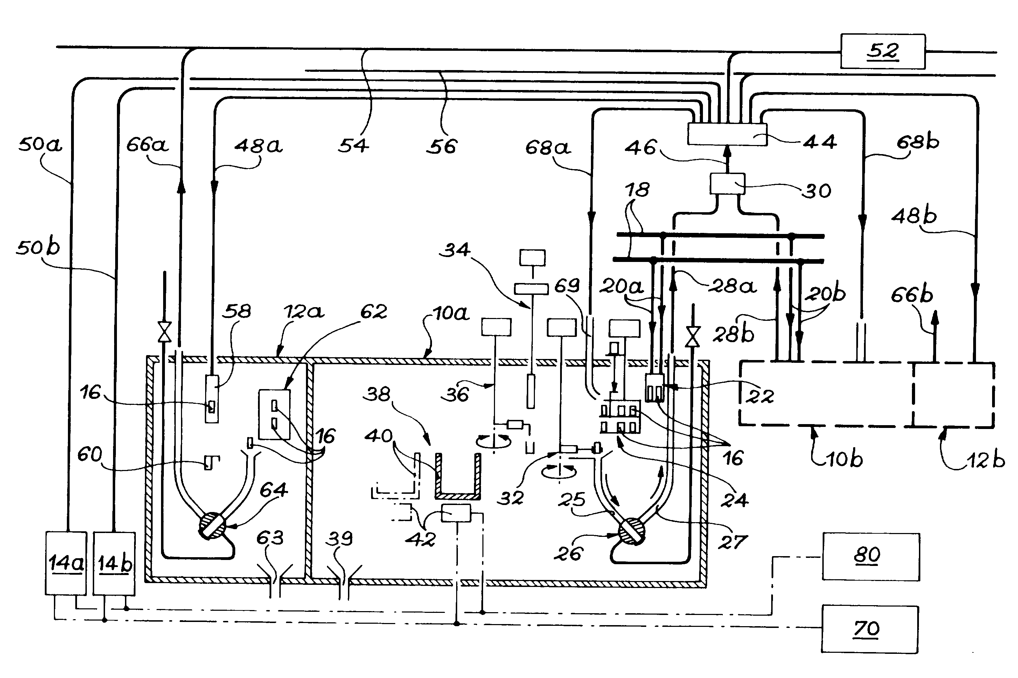

On décrira à présent, à titre d'exemple non limitatif, une forme de réalisation préférentielle de l'invention, en se référant à la figure unique, qui représente de façon schématique une chaîne d'analyses automatisée conforme à l'invention, comportant deux boîtes d'analyses automatiques, deux boîtes d'analyses manuelles et deux appareils de mesure non destructive.

Sur la figure unique, les références 10a et 10b désignent deux boîtes d'analyses automatiques de configuration analogue et les références 12a et 12b désignent deux boîtes d'analyses manuelles, également de configuration analogue, associées respectivement aux boîtes d'analyses automatiques 10a et 10b.

Les boîtes d'analyses automatiques 10a et 10b ainsi que les boîtes d'analyses manuelles 12a et 12b comportent des enceintes de confinement assurant une étanchéité complète vis-à-vis de la migration de la contamination vers l'extérieur. Ces enceintes sont entourées de blindage, par exemple en plomb, assurant une protection du personnel vis-à-vis de l'irradiation.

Les analyses effectuées dans les boîtes d'analyses automatiques 10a et 10b sont des analyses chimiques dont la mise en oeuvre est automatisée. Au contraire, les analyses effectuées dans les boîtes d'analyses manuelles 12a et 12b sont des analyses chimiques dont la mise en oeuvre n'est ou ne peut pas être automatisée. Les analyses effectuées dans les boîtes d'analyses 10a et 12a d'une part et dans les boîtes d'analyses 10b et 12b d'autre part peuvent être identiques ou différentes, selon l'application envisagée.

La chaîne d'analyses illustrée sur la figure unique comprend également deux appareils 14a et 14b permettant d'effectuer des mesures non destructives telles que des mesures physiques. Ces mesures non destructives sont généralement des mesures physiques de natures différentes.

Les analyses qui sont effectuées dans les boîtes automatiques 10a et 10b et manuelles 12a et 12b ainsi que les mesures faites dans les appareils de mesures non destructives 14a et 14b concernent des échantillons de liquide qui sont prélevés en un ou plusieurs points d'une usine nucléaire telle qu'une usine de retraitement de combustible. Ces prélèvements peuvent notamment être effectués au moyen d'installations telles que celle qui est décrite dans le document FR-A-2 675 902. Les échantillons de liquide prélevés sont introduits par ces installations dans des cruchons 16. Ces cruchons 16 sont des récipients étanches fermés par un bouchon vissé et dont la forme cylindrique est étudiée pour permettre leur transfert d'un point à un autre de la chaîne par un réseau de transfert pneumatique dont la conception générale est analogue à celle qui est décrite dans le document FR-A-2 675 582.

Ce réseau de transfert pneumatique comprend tout d'abord une partie amont, par laquelle tous les cruchons 16 en provenance des installations de prélèvement d'échantillons liquides sont acheminés jusqu'à l'une ou l'autre des boîtes d'analyses automatiques 10a et 10b. Sur la figure, cette partie amont du réseau de transfert pneumatique est illustrée par deux conduites principales 18, reliées aux installations de prélèvement (non représentées), et par des dérivations 20a et 20b par lesquelles chacune des conduites principales 18 est reliée respectivement à la boîte d'analyses automatiques 10a et à la boîte d'analyses automatiques 10b. Des mécanismes d'aiguillage (non représentés), par exemple à barillet ou à tiroir, permettent de diriger les cruchons circulant dans les conduites principales 18 vers les dérivations 20a ou 20b, selon la nature des analyses à effectuer ou selon l'encombrement des boîtes d'analyses automatiques 10a et 10b.

L'aménagement intérieur de la boîte d'analyses automatiques 10b étant le même que celui de la boîte d'analyses automatiques 10a, seul l'aménagement de cette dernière boîte d'analyses sera décrit.

A leur entrée dans la boîte d'analyses automatiques 10a, les cruchons 16 introduits par les dérivations 20a chutent par gravité sur des moyens de pesage automatique 22, d'une manière comparable à celle qui est décrite dans le document FR-A-2 675 582. A l'arrivée de chaque cruchon 16, les moyens de pesage automatique 22 émettent un signal représentatif du poids du cruchon. La comparaison de ce signal à un seuil prédéterminé permet donc de savoir si le cruchon 16 admis dans la boîte d'analyses automatiques 10a est plein ou vide.

Selon les résultats de cette comparaison, des moyens de tri (non représentés), associés aux moyens de pesage automatiques 22, permettent soit d'introduire le cruchon 16 dans un dispositif de stockage tampon 24, lorsqu'il est plein, soit d'éliminer le cruchon lorsqu'il est vide. Les moyens de tri peuvent notamment réexpédier les cruchons vides vers un appareillage de destruction des cruchons ou les éjecter dans le fond de la boîte d'analyses automatiques 10a. Dans ce dernier cas, l'automate d'une centrale de commande 80, qui pilote l'ensemble de la chaîne, commande un prélèvement supplémentaire à l'installation de prélèvement correspondante, pour compenser le prélèvement défectueux, et alerte l'opérateur.

Les moyens de tri qui sont associés aux moyens de pesage automatiques 22 sont avantageusement constitués par deux éjecteurs pneumatiques orientés de manière différente, de façon à propulser le cruchon soit dans le dispositif de stockage tampon 24 soit dans le fond de la boîte d'analyses automatiques 10a.

Le dispositif de stockage tampon 24 est constitué par un ensemble modulaire, à un ou plusieurs étages, comprenant par exemple un barillet rotatif pourvu d'alvéoles sur sa périphérie. Plus précisément, ces alvéoles peuvent être ouvertes radialement vers l'extérieur afin qu'un cruchon 16 puisse y être introduit sous l'action d'un jet d'air comprimé, lorsqu'il se trouve en face d'une alvéole. Des motorisations, avantageusement situées à l'extérieur de la boîte d'analyses automatiques 10a, commandent la rotation pas-à-pas du barillet et, éventuellement, sa montée et sa descente lorsque le dispositif de stockage tampon 24 comprend plusieurs niveaux.

L'éjection des cruchons 16 hors du dispositif de stockage tampon 24 se fait également radialement vers l'extérieur, par exemple à l'aide d'un mécanisme commandé par un vérin situé à l'extérieur de la boîte d'analyses automatiques 10a. Cette éjection se fait à un emplacement ou poste fixe, appelé poste d'éjection.

Ce poste d'éjection communique directement avec des moyens 26 pour réexpédier les cruchons 16 dans le réseau de transfert pneumatique, à l'extérieur de la boîte d'analyses automatiques 10a. De façon plus précise, un cruchon éjecté au poste d'éjection du dispositif de stockage tampon 24 se trouve au-dessus de l'embouchure d'une goulotte d'entrée 25, par laquelle ce cruchon peut chuter par gravité dans les moyens 26 pour réexpédier les cruchons.

Ces moyens 26 pour réexpédier les cruchons peuvent notamment comprendre un boisseau tournant, muni d'un réceptacle susceptible d'être placé soit dans le prolongement de la goulotte d'entrée 25 précitée, soit dans le prolongement d'un tube de sortie 27 traversant l'enceinte de la boîte d'analyses automatiques 10a et raccordé sur le réseau de transfert pneumatique à l'extérieur de cette enceinte. Dans cette dernière position, la réexpédition du cruchon s'effectue pneumatiquement. A l'extérieur de la boîte d'analyses, le tube de sortie 27 est prolongé par un tube 28a du réseau de transfert pneumatique pour la boîte d'analyses automatiques 10a et par un tube 28b pour la boîte d'analyses automatiques 10b. Les tubes 28a et 28b sont raccordés sur un aiguillage mécanique 30.

Bien que le poste d'éjection du dispositif de stockage tampon 24 permette d'évacuer directement les cruchons 16 hors de la boîte d'analyses automatiques 10a par les moyens 26 pour réexpédier les cruchons, la majorité d'entre eux ne doit pas suivre ce chemin direct. En effet, le liquide qu'ils contiennent doit faire l'objet d'une ou plusieurs analyses à l'intérieur de la boîte d'analyses automatiques 10a. Cette dernière est donc équipée à cet effet de moyens de traitement automatisés, comprenant un module de préhension 32 qui permet de prélever les cruchons 16 lors de leur éjection du dispositif de stockage tampon 24, au lieu qu'ils ne chutent par gravité dans la goulotte d'entrée 25 des moyens 26 pour réexpédier les cruchons.

Ce module de préhension 32 peut notamment être constitué par une pince de préhension équipée d'un fond escamotable et susceptible d'être déplacée horizontalement entre un poste de préhension et un poste de travail. Lorsqu'elle occupe le poste de préhension, la pince est placée à la verticale du poste d'éjection du dispositif de stockage tampon 24 et immédiatement au-dessus de la goulotte d'entrée 25 des moyens 26 pour réexpédier les cruchons.

Dans le poste de travail de la pince de préhension, les moyens de traitement équipant la boîte d'analyses automatiques 10a permettent d'ouvrir le cruchon 16 placé dans cette pince en en dévissant le couvercle formant bouchon, d'effectuer un ou plusieurs prélèvements et de revisser le couvercle pour refermer le cruchon avant de le réexpédier à l'extérieur de la boîte d'analyses automatiques 10a.

A cet effet, les moyens de traitement qui équipent la boîte d'analyses automatiques 10a comprennent, en plus du module de préhension 32, un module 34 de vissage-dévissage du bouchon équipant les cruchons 16 et un module 36 de prélèvement d'échantillons liquides dans le cruchon. A ces modules s'ajoute une installation d'analyses 38.

Le module 34 de vissage-dévissage du bouchon peut notamment être équipé d'une pince apte à venir serrer le bouchon d'un cruchon tenu par ailleurs par le module de préhension 32. Une rotation de cette pince dans un sens et dans l'autre permet respectivement de visser et de dévisser le bouchon. Un déplacement vertical de la pince entre une position basse de vissage et de dévissage et une position haute permet, dans cette dernière position, de libérer un espace au-dessus du cruchon 16 débouché, afin qu'un ou plusieurs prélèvements de liquide puissent être effectués par le module de prélèvement 36.

Pour sa part, le module de prélèvement 36 peut notamment être équipé d'un embout d'aspiration relié à une pompe à piston au travers d'une garde hydraulique. Cet embout peut être déplacé verticalement afin d'être descendu dans le cruchon tenu par le module de préhension 32 et horizontalement afin d'être amené du poste dans lequel s'effectue le prélèvement à un poste dans lequel l'échantillon prélevé est refoulé dans un récipient 40 de l'appareil d'analyses 38.

Les motorisations commandant les modules 32, 34 et 36 sont avantageusement situées, au moins en partie, à l'extérieur de la boîte d'analyses automatiques 10a.

L'appareil d'analyses automatiques 38 peut comprendre un ou plusieurs béchers ou cuves 40 agencés de façon à pouvoir recevoir un échantillon de liquide à analyser ainsi que des réactifs et des catalyseurs éventuels. Dans le cas où l'appareil d'analyses automatiques 38 comprend plusieurs béchers 40, ceux-ci peuvent être montés sur un plateau rotatif ou disposé en des emplacements fixes au-dessus desquels peut venir se placer successivement l'embout d'aspiration du module de prélèvement 36.

L'appareil d'analyses 38 est pourvu de systèmes automatisés (non représentés) permettant d'injecter les réactifs et les catalyseurs dans chacun des béchers 40, et permettant de vider ces béchers et de les rincer après chaque analyse. Un agitateur, par exemple magnétique, est également placé dans chaque bécher 40. En outre, l'appareil d'analyses 38 comporte des instruments permettant d'effectuer les mesures correspondant aux analyses à effectuer. Les instruments associés à chaque bécher 40 sont schématisés par les blocs 42 sur la figure. Ils sont pilotés par l'automate de la centrale de commande 80, de même que l'ensemble des moyens de traitement équipant les boîtes d'analyses automatiques 10a et 10b. Cela permet notamment de garder sans risque d'erreur la trace de chacun des échantillons et d'assurer la répétabilité des analyses.

En dehors des tubes 28a et 28b et de l'aiguillage 30 sur lequel ces tubes sont raccordés, la partie du réseau de transfert pneumatique située en aval des boîtes d'analyses automatiques 10a et 10b comporte un deuxième aiguillage mécanique 44 situé en aval de l'aiguillage 30 et relié à ce dernier par un tube 46.

Cet aiguillage 44, qui se présente par exemple en pratique sous la forme d'un barillet, permet d'expédier à volonté les cruchons 16 sortant des boîtes d'analyses automatiques 10a et 10b soit vers les boîtes d'analyses manuelles 12a et 12b par des tubes 48a et 48b, soit vers les appareils de mesure non destructive 14a et 14b par des tubes 50a et 50b, soit vers un appareil 52 de recyclage du liquide restant dans les cruchons par un tube 54, soit enfin vers une autre installation telle qu'un laboratoire par un tube 56.

Les appareillages qui équipent intérieurement les boîtes d'analyses manuelles 12a et 12b étant semblables, seul l'appareillage équipant la boîte 12a sera décrit.

Les cruchons 16 qui sont expédiés dans la boîte d'analyses manuelles 12a par le tube 48a pénètrent dans cette boîte par un dispositif d'entrée 58, à bague coulissante, permettant de préserver à tout moment l'étanchéité de la boîte d'analyses manuelles 12a. Ce dispositif d'entrée 58 est conçu de telle sorte que les cruchons 16 chutent automatiquement dans le fond de la boîte d'analyses manuelles 12a.

Lorsqu'un opérateur désire effectuer une analyse sur le liquide contenu dans l'un des cruchons admis dans la boîte d'analyses manuelles 12a, il utilise des pinces de manutention (non représentées) associées à la boîte pour saisir le cruchon 16 correspondant et le placer dans un dispositif 60 de débouchage et de rebouchage des cruchons. Ce dispositif 60 peut être identique au module 34 de vissage-dévissage équipant les boîtes d'analyses automatiques 10a et 10b. De préférence, il s'agit d'un dispositif simplifié et de moindre encombrement dont la commande est faite manuellement à l'aide des pinces de manutention équipant la boîte d'analyses manuelles 12a.

Après ouverture du cruchon 16 considéré, un prélèvement est effectué manuellement à l'aide de pinces dans ce cruchon et l'analyse désirée est faite par l'opérateur à l'aide de moyens appropriés (non représentés) placés à cet effet dans la boîte d'analyses manuelles 12a.

Le bouchon du cruchon 16 est ensuite revissé à l'aide du dispositif 60 et le cruchon refermé est lavé dans un module de lavage 62 également prévu à l'intérieur de la boîte d'analyses manuelles 12a.

Lorsque le lavage est terminé, le cruchon 16 est réexpédié vers l'appareil de recyclage 52 par des moyens 64 pour réexpédier les cruchons. Ces moyens 64 peuvent être avantageusement identiques aux moyens 26 pour réexpédier les cruchons qui équipent les boîtes d'analyses automatiques 10a et 10b. Ils sont reliés à l'appareil de recyclage 52 par un tube 66a, 66b raccordé sur le tube 54 en amont de l'appareil 52.

Les effluents liquides en provenance des appareils d'analyses automatiques 38 dans les boîtes d'analyses automatiques 10a et 10b et des modules de lavage 62 dans les boîtes d'analyses manuelles 12a et 12b sont récupérés par des systèmes d'égouts 39 et 63, puis traités. Il est important d'observer que le volume de ces effluents est aussi faible que possible, notamment pour les boîtes d'analyses automatiques. En effet, ce volume est limité aux faibles volumes prélevés par les modules d'échantillonnage 36, ainsi qu'aux volumes correspondants des réactifs et du liquide de rinçage.

Lorsque les cruchons 16 sont envoyés vers les appareils de mesure non destructive 14a ou 14b par les tubes 50a ou 50b, les mesures sont effectuées dès que le cruchon considéré pénètre à l'intérieur de l'appareil, sans qu'il soit nécessaire de procéder au dévissage du bouchon. Dès que la mesure correspondante est effectuée, le cruchon 16 est réexpédié par le même tube 50a ou 50b dans l'aiguillage 44. A partir de cet aiguillage 44, le cruchon peut être envoyé soit vers l'appareil de recyclage 52 par le tube 54, soit vers un autre laboratoire par le tube 56, soit encore vers l'une ou l'autre des boîtes d'analyses automatiques 10a et 10b par des tubes 68a et 68b. Ces tubes 68a et 68b débouchent dans chacune des boîtes d'analyses automatiques 10a et 10b par un dispositif 69 à bague coulissante analogue au dispositif 58 équipant les boîtes d'analyses manuelles 12a et 12b.

De façon plus précise, les dispositifs à bague coulissante 69 sont disposés de telle sorte que les cruchons 16 ainsi réexpédiés dans les boîtes d'analyses automatiques 10a et 10b soient placés dans les alvéoles du dispositif de stockage tampon 24 par des moyens appropriés tels que des éjecteurs pneumatiques.

En association avec l'aiguillage 44 et avec les dispositifs de stockage tampon 24, la présence des tubes 68a et 68b permet à l'automate de la centrale de commande 80 de choisir, pour chacun des cruchons 16 qui pénètrent dans les boîtes d'analyses 10a et 10b par les dérivations 20a et 20b, l'ordre dans lequel doivent se faire les analyses chimiques et les mesures non destructives. En effet, les cruchons 16 introduits dans le dispositif de stockage tampon 24 peuvent être soit prélevés à la sortie de ce dispositif par le module de préhension 32 pour subir des analyses chimiques, avant d'être réexpédiés vers les appareils de mesure non destructive 14a et 14b pour subir des mesures physiques, soit envoyés directement vers ces appareils de mesure non destructive par les moyens 26 pour réexpédier les cruchons. Dans ce dernier cas, le retour des cruchons dans le dispositif de stockage tampon 24 par les tubes 68a et 68b permet d'effectuer ensuite les analyses chimiques en prélevant les cruchons à la sortie du dispositif de stockage tampon, à l'aide du module de préhension 32.

Les cruchons 16 fermés qui sont réexpédiés vers l'appareil de recyclage 52 ont donc subi les différentes analyses et mesures non destructives souhaitées dans les boîtes d'analyses automatiques 10a et 10b, dans les boîtes d'analyses manuelles 12a et 12b et/ou dans les appareils de mesure non destructive 14a et 14b. Ces cruchons contiennent le reste de liquide non utilisé au cours des analyses chimiques.

Comme l'illustre très schématiquement la figure, l'ensemble des modules équipant les boîtes d'analyses automatiques 10a, 10b et manuelles 12a, 12b, les appareils 14a, 14b de mesure non destructive, l'appareillage de recyclage 52, ainsi que le réseau de transfert pneumatique de la chaîne d'analyses selon l'invention sont pilotés à 100 % par l'automate de la centrale de commande 80. Comme on l'a déjà mentionné, cet automate pilote également les appareils d'analyses automatiques 38 qui équipent les boîtes d'analyses automatiques. Par conséquent, l'ensemble des opérations depuis le départ des cruchons vers les appareils d'analyses jusqu'à l'analyse complète est automatisé et enchaîné.

Enfin, il est à noter que l'automate de la centrale de commande 80 gère l'ensemble des opérations de la chaîne automatisée au travers d'un réseau informatique permettant de remonter les données numériques et alphanumériques telles que des courbes depuis les ordinateurs (non représentés) associés aux différents appareils d'analyses jusqu'à une console de gestion centralisée 70 permettant de suivre et de gérer l'ensemble des opérations.

## Revendications

1. Chaîne d'analyses automatisée, comprenant :
- au moins une boîte d'analyses (10a,10b) apte à recevoir des cruchons fermés (16) contenant un liquide à analyser ;
- un réseau de transfert pneumatique (18,54,56) apte à transférer automatiquement les cruchons dans la boîte d'analyse ; et
- une unité centrale de commande (80) pilotant le réseau de transfert pneumatique ;
caractérisée par le fait que chaque boîte d'analyses comporte :
- des moyens de traitement automatisés (32,34,36,38), pilotés par l'unité centrale de commande (80), pour ouvrir les cruchons, y effectuer des prélèvements, analyser ces prélèvements et refermer les cruchons, ainsi que des premiers moyens (26) pour réexpédier les cruchons dans le réseau de transfert pneumatique (54) ; et
- des moyens (24) de stockage des cruchons, pilotés par l'unité centrale de commande (80) et munis d'un poste d'éjection communiquant directement avec les premiers moyens (26) pour réexpédier les cruchons ;
et par le fait que les moyens de traitement comprennent des moyens de préhension (32) aptes à prélever, puis à réintroduire les cruchons à la sortie du poste d'éjection.

2. Chaîne d'analyses selon la revendication 1, caractérisée par le fait que chaque boîte d'analyses (10a,10b) comporte de plus des moyens (22) de pesage automatique des cruchons à leur arrivée dans la boîte d'analyses, et des moyens de tri, pilotés par l'unité centrale de commande (80), en réponse à des signaux délivrés par les moyens de pesage, aptes à introduire automatiquement les cruchons pleins dans les moyens de stockage (24) et à éliminer les cruchons vides.

3. Chaîne d'analyses selon l'une quelconque des revendications 1 et 2, caractérisée par le fait qu'elle comprend de plus au moins un appareil (14a,14b) de mesure non destructive relié par le réseau de transfert pneumatique aux premiers moyens (26) pour réexpédier les cruchons et à un poste d'alimentation directe des moyens de stockage (24), équipant chaque boîte d'analyses (10a,10b).

4. Chaîne d'analyses selon l'une quelconque des revendications précédentes, caractérisée par le fait qu'elle comprend de plus un appareillage (52) de recyclage de liquide restant dans les cruchons, relié par le réseau de transfert pneumatique aux premiers moyens (26) pour réexpédier les cruchons.

5. Chaîne d'analyses selon les revendications 3 et 4 combinées, caractérisée par le fait que le réseau de transfert pneumatique comprend des moyens d'aiguillage (44) des cruchons, aptes à diriger alternativement les cruchons en provenance des premiers moyens (26) pour réexpédier les cruchons vers l'appareillage de recyclage (52) et vers l'appareil (14a,14b) de mesure non destructive, et aptes à diriger alternativement les cruchons en provenance de l'appareil (14a,14b) de mesure non destructive vers le poste d'alimentation directe et vers l'appareillage de recyclage (52).

6. Chaîne d'analyses selon l'une quelconque des revendications précédentes, caractérisée par le fait qu'elle comprend de plus au moins une boîte d'analyses manuelles (12a,12b) comportant des moyens (60) d'ouverture et de fermeture des cruchons et des deuxièmes moyens (64) pour réexpédier les cruchons dans le réseau de transfert pneumatique (54).

7. Chaîne d'analyses selon les revendications 5 et 6 combinées, caractérisée par le fait que les moyens d'aiguillage (44) sont également aptes à diriger les cruchons en provenance des premiers moyens (26) pour réexpédier les cruchons vers la boîte d'analyses manuelles (12a,12b).

8. Chaîne d'analyses selon l'une quelconque des revendications 6 et 7, caractérisée par le fait que la boîte d'analyses manuelles (12a,12b) comporte de plus des moyens (62) de lavage et de rinçage des cruchons.

## Patentansprüche

1. Automatische Aufeinanderfolge von Analyse-vorrichtungen, umfassend:
- Wenigstens ein Analysegehäuse (10a,10b) zur Aufnahme geschlossener Behälter (16), die eine zu analysierende Flüssigkeit enthalten;
- ein pneumatisches Transfernetz (18,54,56), um die Behälter automatisch in das Analysegehäuse zu befördern; und
- eine zentrale Steuereinheit (80), die das pneumatische Transfernetz steuert;
**dadurch gekennzeichnet,** daß jedes Analysengehäuse umfaßt:
- automatische Bearbeitungseinrichtungen (32,34,36,38), gesteuert durch die zentrale Steuereinheit (80), um die Behälter zu öffnen, Entnahmen aus diesen Behältern vorzunehmen, diese Entnahmen zu analysieren und die Behälter wieder zu verschließen, sowie erste Einrichtungen (26), um die Behälter in dem pneumatische Transfernetz (54) weiterzubefördern; und
- Speichereinrichtungen (24) der Behälter, gesteuert durch die zentrale Steuereinheit (80) und ausgestattet mit einer Auswurfstation, die direkt mit den ersten Einrichtungen (26) zur Weiterbeförderung der Behälter verbunden ist;
und dadurch, daß die Bearbeitungseinrichtungen Greifeinrichtungen (32) umfassen, die fähig sind, Behälter zu entnehmen und dann am Ausgang der Auswurfstation wiedereinzuspeisen.

2. Automatische Aufeinanderfolge von Analyse-vorrichtungen nach Anspruch 1, dadurch gekennzeichnet, daß jedes Analysegehäuse (10a,10b) außerdem Einrichtungen (22) zum automatischen Wiegen der Behälter bei ihrem Eintreffen in dem Analysegehäuse umfaßt, und Sortiereinrichtungen, gesteuert durch die zentrale Steuereinheit (80) als Reaktion auf Signale, geliefert durch die Wiegeeinrichtungen, fähig die vollen Behälter automatisch in die Speichereinrichtungen (24) einzuspeisen und die leeren Behälter zu entfernen.

3. Automatische Aufeinanderfolge von Analyse-vorrichtungen nach einem der Ansprüche 1 und 2, dadurch gekennzeichnet, daß sie außerdem wenigstens eine zerstörungsfreie Meßvorrichtung (14a,14b) umfaßt, die durch das pneumatische Transfernetz mit den ersten Einrichtungen (26) verbunden ist, um die Behälter weiterzubefördern, und mit einer Direktversorgungsstation der Speichereinrichtungen (24), mit der jedes Analysegehäuse (10a,10b) ausgestattet ist.

4. Automatische Aufeinanderfolge von Analyse-vorrichtungen nach einem der vorangehenden Ansprüche, dadurch gekennzeichnet, daß sie außerdem eine Recyclingeinrichtung (52) für die Restflüssigkeit in den Behältern umfaßt, die durch das pneumatischen Transfernetz mit den ersten Einrichtungen (26) zur Weiterbeförderung der Behälter verbunden ist.

5. Automatische Aufeinanderfolge von Analyse-vorrichtungen nach einem der Ansprüche 3 und 4 kombiniert, dadurch gekennzeichnet, daß das pneumatische Transfernetz Verzweigungseinrichtungen (44) der Behälter umfaßt, die fähig sind, abwechselnd die von den ersten Einrichtungen (26) kommenden Behälter zu leiten, um die Behälter weitzubefördern zur Recyclingeinrichtung (52) und zur zerstörungsfreien Meßvorrichtung (14a,14b), und fähig sind, abwechselnd die von der zerstörungsfreien Meßvorrichtung (14a,14b) kommenden Behälter zur Direktversorgungsstation und zur Recyclingeinrichtung (52) zu leiten.

6. Automatische Aufeinanderfolge von Analyse-vorrichtungen nach einem der vorangehenden Ansprüche, dadurch gekennzeichnet, daß sie außerdem wenigstens ein manuelles Analysegehäuse (12a,12b) mit Einrichtungen (60) zum Öffnen und Schließen der Behälter umfaßt, und zweite Einrichtungen (64), um die Behälter in dem pneumatischen Transfernetz (54) weiterzubefördern.

7. Automatische Aufeinanderfolge von Analyse-vorrichtungen nach einem der Ansprüche 5 und 6 kombiniert, dadurch gekennzeichnet, daß die Verzweigungseinrichtungen (44) auch fähig sind, die von den ersten Einrichtungen (26) kommenden Behälter zu leiten, um die Behälter weiterzubefördern zu dem manuellen Analysegehäuse (12a,12b).

8. Automatische Aufeinanderfolge von Analyse-vorrichtungen nach einem der Ansprüche 6 und 7, dadurch gekennzeichnet, daß das manuelle Analysegehäuse (12a,12b) außerdem Wasch- und Spüleinrichtungen (62) der Behälter umfaßt.

## Claims

1. Automated analysis chain having:
- at least one analysis unit (10a, 10b) able to receive sealed jugs (16) containing a liquid to be analyzed;
- a pneumatic transfer system (18, 54, 56) able to automatically transfer the jugs into the analysis unit; and
- a central control unit (80) controlling the pneumatic transfer system;
characterized in that each analysis unit comprises:
- automated processing means (32, 34, 36, 38) controlled by the central control unit (80) for opening the jugs, performing sampling operations therein, analyzing said samples and resealing the jugs, as well as first means (26) for redespatching the jugs to the pneumatic transfer system (54);
- means (24) for the storage of the jugs controlled by the central control unit (80) and provided with an ejection station communicating directly with the first jug redespatch means (26); and in that the processing means incorporate gripping means (32) able to sample and then reintroduce the jugs at the exit from the ejection station.

2. Analysis chain according to claim 1, characterized in that each analysis unit (10a, 10b) also comprises means (22) for the automatic weighing of the jugs when they enter the analysis unit, and sorting means, controlled by the central control unit (80), in response to signals supplied by the weighing means and able to automatically introduce the full jugs into the storage means (24) and eliminate the empty jugs.

3. Analysis chain according to either of the claims 1 and 2, characterized in that at least one non-destructive measuring apparatus (14a, 14b) is connected by the pneumatic transfer system to the first jug redespatch means (26) and to a station for the direct supply of the storage means (24) equipping each analysis unit (10a, 10b).

4. Analysis chain according to any one of the preceding claims, characterized in that an apparatus (52) for recycling liquid remaining in the jugs is connected by the pneumatic transfer system to the first jug redespatch means (26).

5. Analysis chain according to claims 3 and 4 combined, characterized in that the pneumatic transfer system comprises jug switching means (44) able to alternately direct the jugs from the first jug redespatch means (26) to the recycling apparatus (52) and to the non-destructive meausuring apparatus (14a, 14b) and able to alternately direct the jugs from the non-destructive measuring apparatus (14a, 14b) to the direct supply station and to the recycling apparatus (52).

6. Analysis chain according to any one of the preceding claims, characterized in that there is also at least one manual analysis unit (12a, 12b) having means (60) for opening and closing the jugs and second means (64) for the redespatch of the jugs to the pneumatic transfer system (54).

7. Analysis chain according to claims 5 and 6 combined, characterized in that the switching means (44) are also able to direct the jugs from the first jug redespatch means (26) to the manual analysis unit (12a, 12b).

8. Analysis chain according to either of the claims 6 and 7, characterized in that the manual analysis unit (12a, 12b) also has jug washing and rinsing means (62).
